Europäisches Patentamt

European Patent Office

Office européen des brevets

(19)

(11) EP 1 457 778 A1

(12) DEMANDE DE BREVET EUROPEEN

(43) Date de publication:
15.09.2004 Bulletin 2004/38

(51) Int Cl.7: G01N 33/24, E21B 49/00

(21) Numéro de dépôt: 04290476.3

(22) Date de dépôt: 20.02.2004

(84) Etats contractants désignés:
AT BE BG CH CY CZ DE DK EE ES FI FR GB GR
HU IE IT LI LU MC NL PT RO SE SI SK TR
Etats d'extension désignés:
AL HR LT LV MK

(30) Priorité: 11.03.2003 FR 0303073

(71) Demandeur: Institut Français du Pétrole
92852 Rueil-Malmaison Cedex (FR)

(72) Inventeurs:
• Herzhaft, Benjamin
  92150 Suresnes (FR)
• Ropars, Marcel
  91120 Palaiseau (FR)
• Huard, Thierry
  92120 Montrouge (FR)
• Neau, Laurent
  92500 Rueil-Malmaison (FR)

(54) Méthode et dispositif d'analyse du co2 contenu dans un fluide de forage

(57) L'invention concerne une méthode d'estimation de la quantité de $CO_2$ présente dans une formation géologique comportant les étapes suivantes:

- on traverse la formation par un puits foré depuis la surface,
- on contacte la formation avec un fluide qui circule de la formation à la surface,
- on prélève à la surface une quantité déterminée de fluide en retour pour le tranférer dans une cellule,

- on mesure le pH de la quantité de fluide dans la cellule,
- on ajoute une quantité déterminée de produit acidifiant au fluide pour régler le pH à une valeur inférieure à 4,
- on mesure le taux de $CO_2$ du gaz contenu dans la cellule après l'étape d'acidification.

L'invention concerne également un dispositif pour la mise en oeuvre de la méthode.

FIG.4

**Description**

La présente invention concerne le domaine de la surveillance géologique d'un réservoir souterrain, ou de couches géologiques traversées par un forage. La surveillance concerne en particulier, l'analyse en continu, ou en pseudo continu, de la teneur en $CO_2$ du fluide en contact avec le réservoir ou les couches, c'est-à-dire le fluide de forage, ou plus généralement un fluide de puits.

Une analyse précise, et en continu, du $CO_2$ présent dans les boues, ou fluides, de forage représente un atout considérable pour des opérateurs pétroliers. En effet, une bonne détection de la quantité de $CO_2$ dans le réservoir lors du forage s'avère d'une importance capitale pour des raisons économiques ou de sécurité. Une trop grande quantité de $CO_2$ dans l'effluent du réservoir peut générer des surcoûts lors de la mise en production, ou même conduire à l'abandon de l'exploitation du puits. Actuellement, les mesures réalisées a posteriori sur des échantillons de roche réservoir prélevés dans le puits ne sont pas suffisamment sécurisantes, et une autre technique permettant une réponse rapide en cours de forage en apportant l'information recherchée, apparaît indispensable.

On connaît des installations d'analyse des gaz contenus dans le fluide de forage, mais aucune ne permet les mesures précises sur le $CO_2$, comme selon la présente invention.

Ainsi, la présente invention concerne une méthode d'estimation de la quantité de $CO_2$ présente dans une formation géologique comportant les étapes suivantes:

- on traverse ladite formation par un puits foré depuis la surface,

- on contacte ladite formation avec un fluide qui circule de la formation à la surface,

- on prélève à la surface une quantité déterminée de fluide en retour pour le tranférer dans une cellule,

- on mesure le pH de ladite quantité de fluide dans la cellule,

- on ajoute une quantité déterminée de produit acidifiant audit fluide pour régler le pH à une valeur inférieure à 4,

- on mesure le taux de $CO_2$ du gaz contenu dans la cellule après l'étape d'acidification,

- on calcule la quantité de $CO_2$ contenue dans la formation géologique à partir de la mesure précédente du $CO_2$.

Selon l'invention, on peut prendre en compte la quantité de carbonate apportée: par les roches de la formation géologique, et/ou par des additifs de formulation du fluide de forage.

On peut règler à environ 2, le pH de la quantité de fluide.

On peut tranférer le gaz par balayage du volume interne de la cellule par un gaz inerte.

On peut prendre en compte les additifs de formulation de la boue en effectuant la méthode de mesure du $CO_2$ sur un volume déterminé de fluide initial, c'est-à-dire avant contact avec la formation.

La fréquence de prélèvement peut être déterminée en fonction du débit de circulation du fluide.

L'invention concerne aussi un dispositif d'estimation de la quantité de $CO_2$ présente dans une formation géologique traversée par un puits dans lequel un fluide, par exemple de forage, circule entre ladite formation et la tête du puits en surface. Le dispositif comporte des moyens de prélèvement d'une quantité déterminée de fluide de retour à la tête du puits, une cellule servant de réceptacle de ladite quantité de fluide, des moyens de mesure du pH dans ladite cellule, des moyens de balayage par un gaz inerte du volume interne de la cellule, des moyens d'injection d'un produit acidifiant dans ladite cellule, des moyens de mesure de la quantité de $CO_2$ contenue dans le volume interne de la cellule.

Des moyens de réglage peuvent contrôler les moyens d'injection d'acide en fonction de la mesure du pH.

Les moyens de mesure de la quantité de $CO_2$ peuvent comporter une cellule infrarouge, ou une cellule de mesure de la conductivité thermique.

Des moyens de commandes peuvent effectuer les étapes suivantes, à une fréquence déterminée en fonction du débit de fluide: -prélèvement d'une quantité de fluide; -mesure du pH; -injection d'une quantité d'acide; -balayage du volume de la cellule; -mesure de $CO_2$; -vidange de la cellule.

Le dispositif peut comprendre des moyens de mesure de la pression interne à ladite cellule.

Le dispositif peut comprendre des moyens de régulation de la température de ladite cellule.

La présente invention sera mieux comprise et ses avantages apparaîtront plus clairement à la lecture de la description suivante d'exemples de réalisation, nullement limitatifs, illustrés par les figures ci-après annexées, parmi lesquelles:

- La figure 1 montre la répartition des espèces carbonatées en fonction du pH;

- La figure 2 montre l'interaction d'un fluide de forage à base d'eau sur une composition gazeuse contenant du $CO_2$;

- La figure 3 montre l'interaction d'un fluide de forage à base d'huile sur une composition gazeuse contenant du $CO_2$;

- La figure 4 illustre un exemple de réalisation d'un dispositif selon l'invention.

La présente invention permet d'avoir accès aux mesures de toute la quantité de $CO_2$ présente dans une formation géologique. Ces mesures sont effectuées sur le fluide vecteur en relation entre la formation géologique et la surface du sol. Le fluide de forage remonte en circulation dans le puits foré en entrainant le $CO_2$ du fond vers la surface. En faisant dégazer le fluide de forage de retour à la surface, naturellement ou artificiellement, on peut mesurer le $CO_2$ dégazé, mais l'interaction de ce fluide sur le $CO_2$ fausse considérablement les mesures d'estimation de la quantité de $CO_2$ en place dans la formation géologique.

En effet, cela est clairement constaté en étudiant le comportement des mélanges gazeux de $CO_2$ en présence des boues de forage.

Les boues de forage (fluide à base d'eau ou fluide à base d'huile) sont fabriquées en surface à un pH supérieur à 8. La formulation des boues comprend presque systématiquement des quantités importantes de sels (NaCl, $CaCl_2$), et éventuellement des carbonates ($CaCO_3$) pour ajuster la masse volumique du fluide. Le tableau suivant montre un exemple de formulation typique de fluide de forage à l'huile:

| COMPOSANTS | MASSE (g) |
|---|---|
| Huile | 470 |
| Réducteur de filtrat | 6 |
| Emulsifiant | 18 |
| Agent mouillant | 2,9 |
| Chaux | 20 |
| Argile | 15 |
| Viscosifiant | 4 |
| Saumure | 322 |
| $CaCO_3$ (alourdissant) | 260 |

Comme le montre le tableau ci-dessus, la formulation d'une boue à l'huile est un mélange émulsionné aqueux complexe, fortement salin. Cette salinité a un impact sur la solubilité des gaz, en particulier le $CO_2$.

Une boue de forage, en contact avec du $CO_2$ gazeux établit plusieurs équilibres chimiques.

Tout d'abord, le $CO_2$ va se solubiliser partiellement dans la boue selon la loi de Henry, et un équilibre va s'installer entre le $CO_2$ gazeux et le $CO_2$ dissous selon la loi : $[CO_2 aqueux] = K \times PCO_2$, K étant la constante de Henry (dépendant de la formulation de la boue) et $PCO_2$ la pression partielle en $CO_2$ dans le ciel gazeux en contact avec la boue.

Egalement, du fait du pH de la boue, un équilibre va s'établir entre les espèces $CO_2$ aqueux, $HCO_3^-$, $CO_3^{2-}$ selon les formules suivantes:

$$CO_2 \text{ aqueux} + H_2O \leftrightarrow HCO_3^- + H^+ \quad pKa = 6,4$$

$$HCO_3^- \leftrightarrow CO_3^{2-} + H^+ \quad pKa = 10,3$$

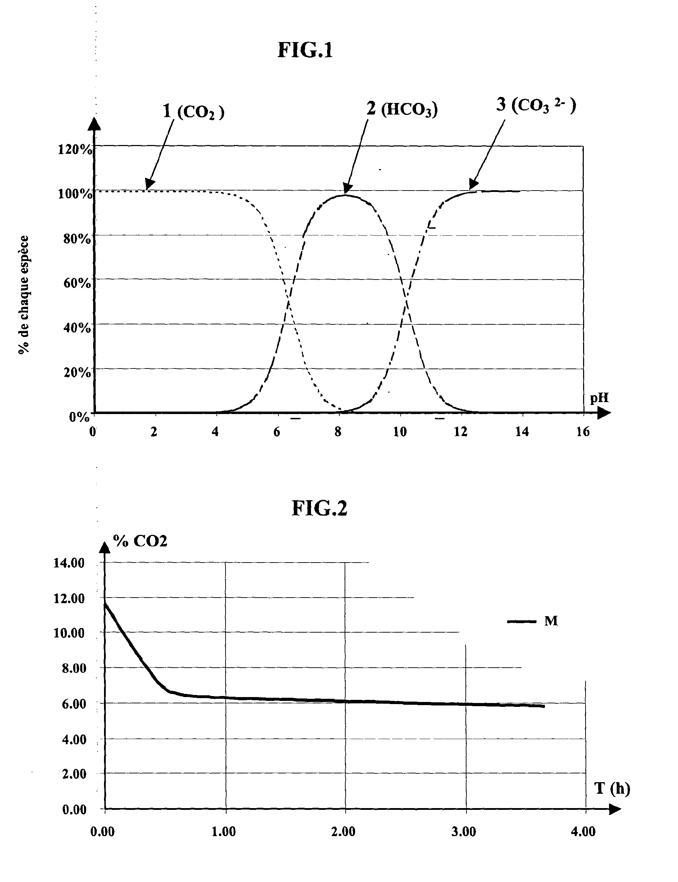

La figure 1 montre les pourcentages des différents composés à base de $CO_2$ en fonction du pH. Les courbes 1, 2 et 3 représentent respectivement les composés: $CO_2$, $HCO_3^-$, et $CO_3^{2-}$.

Outre les propriétés physiques de solubilité du $CO_2$, des propriétés chimiques sont apportées à la solution par le carbonate de calcium selon les réactions suivantes:

- Solubilité des carbonates : $\downarrow CaCO_3 \leftrightarrow Ca^{2+} + CO_3^{2-}$ \quad pKs= 8,3
- Réaction des carbonates avec le $CO_2$ dissous selon :

$$CO_2 \text{ aqueux} + H_2O + CaCO_3 \leftrightarrow Ca(HCO_3)_2$$

Cette dernière réaction est fortement déplacée vers la droite pour les pH élevés et se poursuivra tant qu'il y aura du carbonate de calcium disponible dans la formulation. Du fait du pH élevé de certaines boues de forage et de la présence d'ions $Ca^{2+}$, l'équilibre va avoir tendance à se déplacer vers la formation de carbonates et d'hydrogénocarbonates $Ca(HCO_3)_2$.

Une boue de forage a donc tendance à fonctionner comme une "pompe à $CO_2$".

La figure 2 montre la variation, en fonction du temps T (heure), de la composition d'un ciel gazeux, initialement à 13% en $CO_2$ dans de l'air, mis en contact avec une boue à l'eau de pH = 8,5, et de formulation ci-dessous:

| COMPOSANTS | MASSE (g) |
|---|---|
| Eau | 1000 |
| Argile | 30 |
| Xanthane | 2,5 |
| CMC | 3 |
| Viscosifiant | 5 |
| NaCl | 30 |
| Barytine (alourdissant) | 210 |

On peut voir que très rapidement (moins d'une heure), un équilibre s'établit après la transformation du $CO_2$ en $HCO^{3-}$.

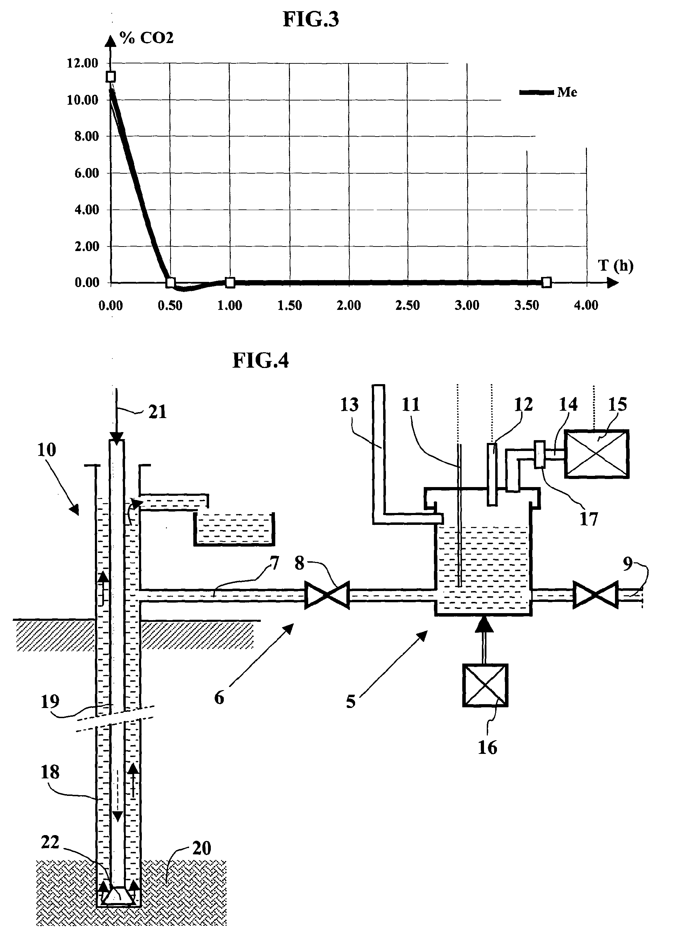

Dans le cas d'une boue à l'huile pH = 10,5 (figure 3), dans les mêmes conditions de ciel gazeux, l'intégralité du $CO_2$ a été consommé par la boue. Aucun équilibre de solubilité n'apparaît, ce qui montre que le $CO_2$ est présent sous forme de carbonates ou d'hydrogénocarbonates en solution dans la boue.

Pour effectuer une mesure du $CO_2$ transporté par la boue de forage, il faut donc s'attacher à doser les espèces carbonates et hydrogénocarbonates présentes dans la boue, notamment en déplaçant les équilibres chimiques.

Pour cela, selon l'invention, une acidification forte du milieu permet la transformation des différentes espèces en $CO_2$ gazeux. La libération du $CO_2$ peut être réalisée en acidifiant fortement la solution avec une injection d'acide concentré jusqu'à atteindre, au moins un pH inférieur à 4, et de préférence un pH environ de 2.

Au pH de 2, toutes les espèces carbonatées auront été transformées en $CO_2$ gazeux dont on peut mesurer la teneur à l'aide d'un capteur infrarouge, ou par conductivité thermique. L'augmentation de la pression dans la cellule de mesure peut également être une mesure indirecte de la quantité de $CO_2$ libéré.

La figure 4 montre schématiquement le principe du dispositif de mesure. Le dispositif comprend une cellule 5 fermée reliée à la tête de puits 10 par des moyens de prélèvement 6 comprenant une conduite 7 et des moyens de distribution 8, par exemple une pompe et/ou des vannes. La cellule 5 comprend également des moyens d'évacuation 9 du volume prélevé dans la cellule. La cellule comporte des moyens de mesure 11 du pH, de préférence mesuré en continu, et éventuellement des moyens de mesure 12 de la pression interne à la cellule. Des moyens de balayage par un gaz, inerte de préférence, comportent un conduit 13 d'amené du gaz inerte dans la cellule et un conduit 14 de sortie du gaz contenu dans le "ciel" de la cellule, au-dessus du volume de boue prélevé. Le conduit de sortie 14 dirige ce gaz vers un analyseur 15 de $CO_2$, par exemple un capteur infrarouge, ou autre dispositif de mesure connu. Une pompe à acide 16 permet l'injection d'un acide fort dans la cellule, selon un débit et un volume contrôlés en fonction de la vitesse d'acidification désirée et du suivi de l'évolution du pH donné par le capteur 11. Des moyens de contrôle et de régulation de la température (non représentés) de la cellule peuvent compléter le dispositif.

Le prélèvement de l'échantillon de boue de forage directement en tête de puits peut se faire automatiquement, ou être commandé par un opérateur. Les prélèvements peuvent être séquentiels, à une fréquence qui sera fonction notamment du débit de fluide de forage. L'ensemble des capteurs, des analyseurs, des moyens commandés sont reliés à un ordinateur de commande (non représenté) de tous ces organes: vannes, moteurs, capteurs, injecteurs,...

Une membrane 17 permettant d'assécher le gaz est placée avant le capteur 15, de façon à éliminer les traces d'eau pouvant être contenues dans ce ciel gazeux.

La figure 1 montre schématiquement une couche géologique 20 traversée, partiellement par un forage 18 exécuté par une garniture de forage 19. Le fluide de forage injecté en 21 remonte de l'outil de forage 22, auprès de la formation 20, vers la surface, selon la symbolisation des flèches.

L'augmentation de la pression dans la cellule peut être corrélée à la production de $CO_2$.

Exemple de mode opératoire:

- Prélèvement automatique à la tête de puits d'un volume déterminé d'échantillon de boue de forage;

- Déclenchement du flux de gaz inerte jusqu'au capteur infrarouge;

- Injection d'acide à l'aide de la pompe seringue et suivi du pH;

- Mesure du $CO_2$ en continu à l'aide du capteur infrarouge, ou de conductivité thermique;

- Arrêt de la mesure après acidification au pH = 2.

La mesure $M_{infrarouge}$ ainsi effectuée donne un équivalent $CO_2$ de toutes les espèces, y compris carbonatées et hydrogénocarbonatées, présentes dans la boue de forage. A cette valeur, il convient de retirer la quantité $M_{densité}$ de $CO_2$ correspondant à la quantité de carbonate initialement présente dans la boue de forage, notamment due aux additifs destinés à ajuster sa masse volumique. Cette valeur peut être connue en réalisant une mesure sur la formulation initiale, sur site ou en laboratoire.

Dans le cas de forage de formations géologiques carbonatées, la connaissance géologique du terrain nous donne une proportion de carbonate dans la zone forée $C_{formation}$. Le volume de déblais contenus dans la boue de forage peut être calculé en considérant le volume du trou, le débit de boue et la vitesse d'avancement, soit V ce volume de déblais par litre de boue.

On aura donc la quantité de carbonate provenant de la formation:

$$Ca_{formation} = V_{boue} \times V \times C_{formation}$$

$M_{formation}$ étant la quantité de $CO_2$ correspondante à la quantité $Ca_{formation}$, la quantité de $CO_2$ de la formation géologique transporté par la boue depuis le fond du puits sera donc égale à : $M_{infrarouge} - M_{densité} - M_{formation}$

Ainsi, la méthode et le dispositif selon l'invention permet une estimation plus vraie de la quantité de $CO_2$ en place dans un gisement souterrain, en prenant en compte l'interaction du fluide de transport de ce gaz, les conditions initiales de la nature de ce fluide, et les opérations de forage dans ce gisement.

**Revendications**

1. Méthode d'estimation de la quantité de $CO_2$ présente dans une formation géologique comportant les étapes suivantes:

   - on traverse ladite formation par un puits foré depuis la surface,

   - on contacte ladite formation avec un fluide de forage de pH supérieur à 8 qui circule de la formation à la surface,

   - on prélève à la surface une quantité déterminée de fluide en retour pour le tranférer dans une cellule,

   - on mesure le pH de ladite quantité de fluide,

   - on ajoute une quantité déterminée de produit acidifiant audit fluide pour régler le pH à une valeur inférieure à 4,

   - on mesure le taux de $CO_2$ du gaz contenu dans la cellule après l'étape d'acidification,

   - on calcule la quantité de $CO_2$ contenue dans la formation géologique à partir de la mesure du $CO_2$.

2. Méthode selon la revendication 1, dans laquelle on prend en compte la quantité de carbonate apportée: par la formation géologique et/ou par des additifs de formulation dudit fluide.

3. Méthode selon l'une des revendications 1 ou 2, dans laquelle on règle le pH à environ 2.

4. Méthode selon l'une des revendications précédentes, dans laquelle on tranfère ledit gaz par balayage du volume interne de la cellule par un gaz inerte.

5. Méthode selon la revendication 2, dans laquelle on prend en compte lesdits additifs en effectuant la méthode de mesure du $CO_2$ sur un volume déterminé de fluide initial, c'est-à-dire avant contact avec la formation.

6. Méthode selon l'une des revendications précédentes, dans laquelle la fréquence de prélèvement est déterminée en fonction du débit de circulation du fluide.

7. Dispositif d'estimation de la quantité de $CO_2$ présente dans une formation géologique traversée par un puits dans lequel un fluide de forage de pH supérieur à 8 circule entre ladite formation et la tête du puits en surface, **caractérisé en ce qu'**il comporte des moyens de prélèvement d'une quantité déterminée de fluide à la tête du puits, une cellule réceptacle de ladite quantité de fluide, des moyens de mesure du pH dans ladite cellule, des moyens de balayage par un gaz inerte du volume interne de la cellule, des moyens d'injection d'un produit acidifiant dans ladite cellule, des moyens de mesure de la quantité de $CO_2$ contenue dans le volume interne de la cellule.

8. Dispositif selon la revendication 7, dans lequel des moyens de réglage contrôlent les moyens d'injection d'acide en fonction de la mesure du pH.

9. Dispositif selon l'une des revendications 7 ou 8, dans lequel les moyens de mesure de la quantité de $CO_2$ comportent une cellule infrarouge ou une cellule de mesure de la conductivité thermique.

10. Dispositif selon l'une des revendications 7 à 9, dans lequel des moyens de commandes effectuent les étapes suivantes, à une fréquence déterminée en fonction du débit de fluide: -prélèvement d'une quantité de fluide; -mesure du pH; -injection d'une quantité d'acide; -balayage du volume de la cellule; -mesure de $CO_2$; -vidange de la cellule.

11. Dispositif selon l'une des revendications 7 à 10, comprenant des moyens de mesure de la pression interne à ladite cellule.

12. Dispositif selon l'une des revendications 7 à 11, comprenant des moyens de régulation de la température de ladite cellule.

## FIG.1

1 ($CO_2$)    2 ($HCO_3$)    3 ($CO_3^{2-}$)

% de chaque espèce

120%
100%
80%
60%
40%
20%
0%

0    2    4    6    8    10    12    14    16

pH

## FIG.2

% CO2

14.00
12.00
10.00
8.00
6.00
4.00
2.00
0.00

— M

0.00    1.00    2.00    3.00    4.00

T (h)

**FIG.3**

**FIG.4**

**Office européen des brevets**

## RAPPORT DE RECHERCHE EUROPEENNE

Numéro de la demande

EP 04 29 0476

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y | US 3 685 345 A (WISE HAROLD L) 22 août 1972 (1972-08-22) * colonne 2, ligne 19-38 * * colonne 4, ligne 45-59 * * revendications 1-3 * * figure 1 * | 1-12 | G01N33/24 E21B49/00 |
| Y | US 5 992 213 A (TARTRE ANDRE) 30 novembre 1999 (1999-11-30) * colonne 1, ligne 9-45 * * colonne 2, ligne 6-29 * * colonne 3-4 * * colonne 5, ligne 3-10 * | 1-12 | |
| Y | PATENT ABSTRACTS OF JAPAN vol. 011, no. 287 (P-617), 17 septembre 1987 (1987-09-17) & JP 62 083647 A (FUJITSU LTD), 17 avril 1987 (1987-04-17) * abrégé * | 1-12 | |
| A | US 5 319 966 A (JACKSON RICHARD E ET AL) 14 juin 1994 (1994-06-14) * colonne 1, ligne 1 - colonne 3, ligne 56 * | 1-12 | **DOMAINES TECHNIQUES RECHERCHES (Int.Cl.7)** G01N E21B |
| A | US 6 289 714 B1 (TARTRE ANDR EACUTE) 18 septembre 2001 (2001-09-18) * colonne 2-4 * * figures 1-3 * | 1-12 | |

Le présent rapport a été établi pour toutes les revendications

| Lieu de la recherche | Date d'achèvement de la recherche | Examinateur |
|---|---|---|
| MUNICH | 12 juillet 2004 | Michalitsch, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 04 29 0476

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-07-2004

| Document brevet cité au rapport de recherche | | Date de publication | Membre(s) de la famille de brevet(s) | | Date de publication |
|---|---|---|---|---|---|
| US 3685345 | A | 22-08-1972 | CA | 929087 A1 | 26-06-1973 |
| US 5992213 | A | 30-11-1999 | US | 6289714 B1 | 18-09-2001 |
| JP 62083647 | A | 17-04-1987 | AUCUN | | |
| US 5319966 | A | 14-06-1994 | AUCUN | | |
| US 6289714 | B1 | 18-09-2001 | US | 5992213 A | 30-11-1999 |

EPO FORM P0460

Pour tout renseignement concernant cette annexe : voir Journal Officiel de l'Office européen des brevets, No.12/82